Europäisches Patentamt

European Patent Office

Office européen des brevets

(19)

(11) Publication number : **0 307 404 B1**

# (12) EUROPEAN PATENT SPECIFICATION

(45) Date of publication of patent specification :
**02.01.92 Bulletin 92/01**

(51) Int. Cl.⁵ : **C07C 43/04, C07C 41/06**

(21) Application number : **87903295.1**

(22) Date of filing : **07.05.87**

(86) International application number :
**PCT/EP87/00241**

(87) International publication number :
**WO 87/07259 03.12.87 Gazette 87/27**

(54) PROCESS FOR PREPARING ALKYL-TERT-BUTYL ETHERS.

(30) Priority : **27.05.86 IT 2057586**

(43) Date of publication of application :
**22.03.89 Bulletin 89/12**

(45) Publication of the grant of the patent :
**02.01.92 Bulletin 92/01**

(84) Designated Contracting States :
**AT BE DE FR GB IT NL SE**

(56) References cited :
**EP-A-01 038 70**
**FR-A- 2 272 064**
**GB-A- 2 116 546**
**US-A-42 425 26**

(73) Proprietor : **SNAMPROGETTI S.p.A.**
**Corso Venezia 16**
**I-20121 Milan (IT)**

(72) Inventor : **PESCAROLLO, Ermanno**
**Via Spezia, 45**
**I-20142 Milan (IT)**
Inventor : **ANCILLOTTI, Francesco**
**Via Agadir, 14/C**
**I-20097 San Donato Milanese (IT)**

(74) Representative : **Roggero, Sergio et al**
**Ing. Barzanò & Zanardo Milano S.p.A. Via**
**Borgonuovo 10**
**I-20121 Milano (IT)**

EP 0 307 404 B1

Jouve, 18, rue Saint-Denis, 75001 PARIS

## Description

The present invention relates to a process for the preparation of alkyl-tert-butyl ethers in the presence of butadiene.

It is known that for the synthesis of said ethers it is not necessary to use high-purity isobutene, but a fraction is enough which contains it, even if at a low concentration.

Suitable olefinic fractions for said syntheses are the $C_4$ cuts outcoming from catalytic cracking, or those obtained from steam cracking, these latter both before and after butadiene extraction.

If as the feed olefinic charges, the $C_4$ fractions from catalytic cracking, or those deriving from steam cracking after butadiene removal are used, and as the alcohols methanol or ethanol are used, in the presence of catalysts constituted by macroporous sulphonated resins, of Amberlyst 15 or Lewatit SPC 108 type, the reaction can be carried out on an industrial scale, according to a wide range of schemes and of operating conditions directed towards the optimization of conversion of either of reactants.

In these cases, a high selectivity, as well as a good catalyst performance is always obtained, and no problems are to be faced as to the products separation and recovery sections.

If the process is carried out with a high-butadiene-content charge (containing from 10 to 70% by weight of butadiene), such as the $C_4$ cut before butadiene extraction, the operating conditions have to be exactly defined to the purpose of recovering more than 98-99% of butadiene. In particular, maintaining a close relationship between temperature and space velocity is imperative, as disclosed in US-A-4 039 590; this ensures a low butadiene conversion, but, to the purpose of obtaining a catalyst performance such as to allow industrial applicability, it is required to work under certain particular measures as to the reactor, and the temperature should not exceed 60°C, as disclosed in GB-A-2 116 546.

EP-A2-103 870 discloses a combined ether and MTBE manufacture with HF alkylation of isobutane, the linear butenes being recovered from the MTBE process, wherein, to prevent HF- and propane loss, the reactant alcohol is stripped of light gases, such as oxygen and nitrogen, prior to etherification.

Even operating under the prior art conditions, butadiene polymerizes in the fractional-distillation section in which tert.butyl ether is separated from the unreacted charge: such polymerization is detrimental to the fractional-distillation run. Polymerization inhibitors, which are efficient within a liquid phase are virtually inoperative within gaseous media.

It has been found, surprisingly, that it is now possible efficiently to prevent noxious polymerization by previously stripping the etherification reactor feedstock from the oxygen dissolved therein.

Oxygen mainly derives from air dissolved in the aliphatic alcohol feedstock (methanol or ethanol), due to shipping and storage conditions. For example, methanol used in synthesizing MTBE, if stored at 20°C under atmospherical pressure (1013,25 mbar), dissolves as much as about 80 ppm weight/weight (w/w) of oxygen and 180 ppm w/w of nitrogen; the presence of this latter, however, does not create any problems.

A certain amount of dissolved oxygen can be occasionally present also in the liquified $C_4$ fraction containing isobutene and isobutadiene, in particular when such a fraction has been stored at low temperatures, at which the vapour pressure of the mixture becomes lower than atmospheric. In general, however, nearly all of the oxygen dissolved in the reaction mixture fed to the etherification reactor is introduced through the alcohol.

The removal of oxygen from alcohol is enough to overcome the problem of polymers formation.

The procedures for oxygen removal are those known in the art for the removal of inert gases dissolved in liquid products, such as, e.g., the use of a suitable stripper, consisting in a distillation column, from the bottom of which the oxygen-free product is obtained, whilst oxygen is discharged as a overhead vent from the reflux accumulator.

Summing-up, the process of the present invention is a process for the preparation of alkyl-tert-butyl ethers by starting from $C_4$ charges containing isobutene and other $C_4$ hydrocarbons, among which butadiene at a concentration, referred to the total charge, comprised within the range of from 10% by weight to 70 by weight ($C_4$ charges coming from catalytic cracking or from steam cracking without butadiene removal) and from aliphatic alcohols preferably selected from methanol and ethanol, and comprises reacting the isobutene contained in the charge with the aliphatic alcohol in one or more reaction sections at a temperature not higher than 60°C in the presence of a macroporous sulphonated resin of Amberlyst 15 or Lewatit SPC 108 type and separating the alkyl-tert-butyl ether from the other unreacted components by distillation, and is characterized in that the alcohol and/or the $C_4$ hydrocarbon charge are/is submitted, before the reaction, to an oxygen removal step, in particular of physical type, of removal of dissolved free oxygen by stripping by outer inert agents, or distillation up to reduce the free oxygen content to 1-2 ppm or less.

In special cases, the oxygen removal may be carried out by stripping by a stream of a suitable inert gas, such as nitrogen, methane, hydrogen, fuel gas, ethane, etc., or mixtures thereof.

In the following examples, some oxygen removal procedures are described, which are supplied to purely exemplifying purposes, and in no way are limitative of the present invention.

## Example 1 (comparative)

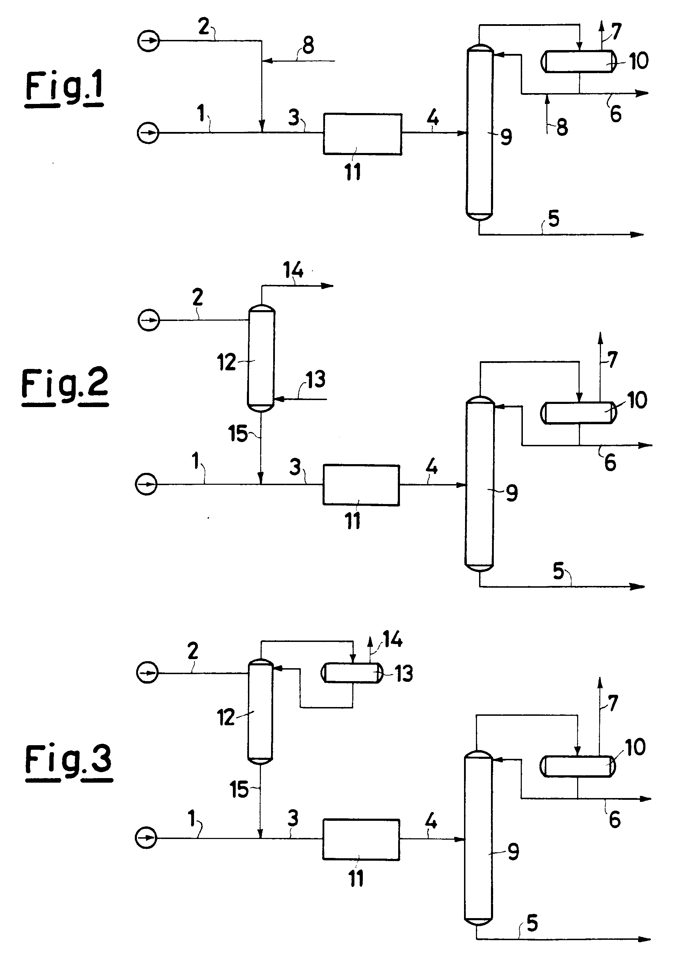

100 parts by weight of an olefinic charge (1), see Figure 1, constituted by a $C_4$ cut from steam cracking before butadiene extraction, and containing on the average 25% by weight of isobutene and 50% by weight of butadiene, are mixed with 15.3 parts by weight of methanol (2) and the resulting charge (3) is fed to reactor (11) containing an ion-exchange resin in the acidic form, of Amberlyst 15 type.

The olefinic cut (1) has a content of dissolved oxygen lower than 1 ppm, whilst methanol, which is stored inside a tank under an air atmosphere, has an oxygen content ranging from 30 to 60 mg/l.

To the stream (2) an alcoholic solution of a polymerization inhibitor is injected (8) in such an amount that in the combined charge (3) the ihnibitor concentration ranges from 60 to 120 ppm. The reaction product (4) containing an average amount of 30.7% of MTBE, which justifies an isobutene conversion of 90%, is fed to a fractionation tray column (9) in such an intermediate point, that the rectification section represents 40% of column. From the top of said fractionation column the not-converted hydrocarbons are removed together with azeotropic methanol (6), from the bottom of the column a stream (5) is removed, which is constituted by practically pure MTBE. The column is operated under a pressure of 4.9 bars, the overhead, feed and bottom temperatures are respectively 41°C, 60°C and 125°C. Oxygen dissolved in stream (4) fed to column (9) accumulates on column top, and is partly eliminated as an overhead vent from reflux accumulator (10); in stream (7), oxygen reaches values comprised within the range of from 3000 to 6000 ppm.

Feed conditions and reflux are such that the pressure drop $\Delta P$, which is established in the column, is of 0.3 bars.

After said column has been operating, in continuous run, for 860 hours under the above disclosed conditions, $\Delta P$ rises up to values more than double of the initial value, while the fractionation capacity results seriously impaired.

An inspection evidences a large extent of formation of polymeric material on the trays of the rectificaton section of the column.

## Example 2 (comparative)

MTBE synthesis and fractionation are carried out as in Example 1, with the difference that the polymerization inhibitor is injected, by the stream (8), Figure 1, also to the column reflux.

The obtained improvement is partial only, in as much as after 2000 running hours, butadiene polymerization on rectification trays block agains column operation.

## Example 3

MTBE synthesis and fractionation are performed under operating conditions analogous to those of Example 1, with the variant (see Figure 2) of the treatment of methanol (2) which contains an amount of dissolved oxygen ranging from 30 to 60 mg/l, and is delivered to the upper section of a stripping column (12), from the bottom of which a stream of an inert gas, such as fuel gas (13), containing an oxygen amount lower than 150 ppm, is sent in countercurrent flow.

The oxygen-depleted methanol, stream (15), is recovered from the bottom of the column, with oxygen levels constantly lower than 1 ppm.

The oxygen-enriched stripping gas (14) is sent to blowdown. The efficaciously of the stripping is also confirmed by the fact that in gaseous stream (7) the oxygen level does not exceed 20 ppm. By resorting to such a measure, column (9) has been operating for 8000 hours with no drawbacks, and an inspection thereof showed the complete absence of polymerization phenomena. The equal find numbers have the same meaning as of Figure 1.

## Example 4

MTBE synthesis and its fractionation are carried out as in preceding Examples, but methanol is now deprived of oxygen by sending stream (2) (Figure 3) to the head of a distillation column (12), from the bottom of which methanol is recovered with an oxygen level lower than 0.5 ppm.

The initially dissolved oxygen is removed as a overhead vent (14) from reflux accumulator (13). The oxygen amount found in the overhead vent (7) is lower than 10 ppm. By so doing, the column showed to be able to operate without drawbacks for more than 8000 hours.

The other find numbers indicate the elements which were already illustrated in Figure 1.

## Claims

1. Process for the preparation of alkyl-tert-butyl ethers by starting from $C_4$ charges containing isobutene and other $C_4$ hydrocarbons, among which butadiene at a concentration, referred to the total charge, comprised within the range of from 10% by weight to 70% by weight and from aliphatic alcohols preferably selected from methanol and ethanol, comprising reacting the isobutene contained in the charge with the aliphatic alcohol in one or more reaction sections at a temperature not higher than 60°C in the presence of a macroporous sulphonated resin of Amberlyst 15 or Lewatit SPC 108 type and separating the alkyl-tert-butyl ether from the other unreacted components by distillation, characterized in that the alcohol and/or the $C_4$ hydrocarbon charge are/is submitted, before the reaction, to an oxygen removal step, in particular of physical type, of removal of dissolved free oxygen by stripping with outer inert agents, or distillation up to reduce the free oxygen content to 1-2 ppm or less.

2. Process according to claim 1, wherein the oxygen removal is of physical type.

3. Process according to claim 2, characterized in that the oxygen removal is carried out by stripping with inert outer agents.

4. Process according to claim 3, characterized in that the inert outer agents are selected from nitrogen, methane, hydrogen, fuel gas, ethane or mixtures thereof.

5. Process according to claim 2, characterized in that the oxygen removal is carried out by distillation, by sending the stream do be deprived of oxygen to the head of a distillation column.


## Revendications

1. Procédé de préparation d'éthers d'alkyle et de tertio-butyle, à partir de charges en $C_4$ contenant de l'isobutène et d'autres hydrocarbures en $C_4$, parmi lesquels du butadiène en une concentration, rapportée à la charge totale, située dans l'intervalle allant de 10 % en poids à 70 % en poids, et d'alcools aliphatiques choisis de préférence parmi le méthanol et l'éthanol, procédé qui consiste à faire réagir l'isobutène contenu dans la charge avec l'alcool aliphatique, en un ou plusieurs lieux de réaction, à une température ne dépassant pas 60° C, en présence d'une résine macroporeuse sulfonée de type Amberlyst 15 ou Lewatit SPC 108, et à séparer par distillation les éthers d'alkyle et de tertio-butyle d'avec les autres composants n'ayant pas réagi, procédé caractérisé en ce que l'alcool et/ou la charge d'hydrocarbures en $C_4$ est/sont soumis, avant la réaction, à une étape d'élimination de l'oxygène, en particulier de type physique, dans laquelle on élimine l'oxygène libre dissous par extraction à l'aide d'agents extérieurs inertes, ou par distillation, jusqu'à réduire la teneur en oxygène libre jusqu'à 1-2 ppm ou moins.

2. Procédé conforme à la revendication 1, dans lequel l'élimination d'oxygène est un processus de type physique.

3. Procédé conforme à la revendication 2, caractérisé en ce que l'élimination d'oxygène est effectuée par extraction à l'aide d'agents extérieurs inertes.

4. Procédé conforme à la revendication 3, caractérisé en ce que les agents extérieurs inertes sont choisis parmi l'azote, le méthane, l'hydrogène, le gaz de chauffage, l'éthane et leurs mélanges.

5. Procédé conforme à la revendication 2, caractérisé en ce que l'élimination d'oxygène est effectuée par distillation, le courant qui doit être débarassé de son oxygène étant envoyé à la tête d'une colonne de distillation.


## Patentansprüche

1. Verfahren zur Herstellung von Alkyl-tert.-butylethern ausgehend von $C_4$-Chargen, welche Isobuten und andere $C_4$-Kohlenwasserstoffe enthalten, unter welchen Butadien in einer Konzentration, bezogen auf die Gesamtcharge, innerhalb des Bereiches von 10 Gew.-% bis 70 Gew.-% vorliegt, und von aliphatischen Alkoholen, welche vorzugsweise unter Methanol und Ethanol ausgewählt sind, umfassend das Umsetzen des in der Charge enthaltenen Isobutens mit dem aliphatischen Alkohol in einem oder mehreren Reaktionsabschnitten bei einer Temperatur von nicht höher als 60°C in Gegenwart eines makroporösen sulfonierten Harzes vom Amberlyst 15- oder Lewatit SPC 108-Typ und Abtrennen des Alkyl-tert.-butylethers von den anderen nicht umgesetzten Komponenten durch Destillation, dadurch gekennzeichnet, daß der Alkohol und/oder die $C_4$-Kohlenwasserstoffcharge vor der Reaktion einem Sauerstoffentfernungsschritt, insbesondere von physikalischer Art, zur Entfernung von gelöstem, freiem Sauerstoff durch Strippen mit äußeren inerten Mitteln oder Destillation bis zu einer Verringerung des Gehaltes an freiem Sauerstoff von 1 bis 2 ppm oder weniger, unterworfen wird (werden).

2. Verfahren nach Anspruch 1, worin die Sauerstoffentfernung von physikalischer Art ist.

3. Verfahren nach Anspruch 2, dadurch gekennzeichnet, daß die Sauerstoffentfernung durch Strippen mit äußeren inerten Mitteln ausgeführt wird.

4. Verfahren nach Anspruch 3, dadurch gekennzeichnet, daß die äußeren inerten Mittel unter Stickstoff, Methan, Wasserstoff, Brenngas, Ethan oder Gemischen hievon ausgewählt sind.

5. Verfahren nach Anspruch 2, dadurch gekennzeichnet, daß die Sauerstoffentfernung durch Destillation ausgeführt wird, indem der von Sauerstoff zu befreiende Strom zum Kopf einer Destillationskolonne geschickt wird.

Fig.1

Fig.2

Fig.3